# EUROPEAN PATENT SPECIFICATION

(11) **EP 1 553 948 B1**
(45) Date of publication and mention of the grant of the patent: **24.08.2011**
(21) Application number: 03795175.3
(22) Date of filing: 10.09.2003
(51) Int. Cl.: A61K 31/506, A61K 31/404, A61K 31/165, A61K 31/166, A61K 31/19, A61K 31/4406, A61P 35/02

(54) **COMBINATION OF IMATNIB AND A HISTONE DEACETYLASE INHIBITOR FOR THE TREATMENT OF LEUKEMIA**
KOMBINATION AUS IMATINIB UND EINEM HISTONDEACETYLASE-HEMMER ZUR BEHANDLUNG VON LEUKÄMIE
COMBINAISON DE IMATINIB ET D'UN INHIBITEUR DE L'HISTONE DEACETYLASE POUR LE TRAITEMENT DE LA LEUCEMIE

(30) Priority: 13.09.2002 US 410286 P; 18.09.2002 US 411344 P
(43) Date of publication of application: 20.07.2005
(73) Proprietor: VIRGINIA COMMONWEALTH UNIVERSITY, Richmond, Virginia 23298-0568 (US); McGuire Va Medical Center 111K, Richmond, VA 23249 (US)
(72) Inventor: DENT, Paul, Glen Allen, VA 23060 (US); GRANT, Steven, Richmond, VA 23298 (US); KRYSTAL, Geoffrey, Richmond, VA 23233 (US); YU, Chunrong, Rochester, MN 55901 (US)
(74) Representative: Krauss, Jan
(86) International application number: PCT/IB2003/004053
(87) International publication number: WO 2004/024160

(56) References cited:
- ROSEE LA P ET AL: "PRECLINICAL EVALUATION OF THE EFFICACY OF STI571 IN COMBINATION WITH A VARIETY OF NOVEL ANTICANCER AGENTS" BLOOD, W.B.SAUNDERS COMPAGNY, ORLANDO, FL, US, vol. 98, no. 11, PART 1, 16 November 2001 (2001-11-16), page 839A XP009010653 ISSN: 0006-4971
- ROSEE LA P ET AL: "INSIGHTS FROM PRE-CLINICAL STUDIES FOR NEW COMBINATION TREATMENT REGIMENS WITH THE BCR-ABL KINASE INHIBITOR IMATINIB MESYLATE (GLEEVEC/GLIVEC) IN CHRONIC MYELOGENOUS LEUKEMIA: A TRANSLATIONAL PERSPECTIVE" LEUKEMIA, MACMILLAN PRESS LTD, US, vol. 16, no. 7, 2002, pages 1213-1219, XP009005469 ISSN: 0887-6924
- MELLINGHOFF I K ET AL: "The emergence of resistance to targeted cancer therapeutics" PHARMACOGENOMICS 2002 UNITED KINGDOM, vol. 3, no. 5, 2002, pages 603-623, XP009022496 ISSN: 1462-2416
- ROSATO ROBERTO R ET AL: "Histone deacetylase inhibitors interact in a highly synergistic manner with TRAIL to induce mitochondrial damage and apoptosis in human leukemia cells" PROCEEDINGS OF THE AMERICAN ASSOCIATION FOR CANCER RESEARCH ANNUAL, vol. 43, March 2002 (2002-03), page 701 XP001156354 93rd Annual Meeting of the American Association for Cancer Research;San Francisco, California, USA; April 06-10, 2002, March, 2002 ISSN: 0197-016X
- ALMENARA JORGE ANTONIO ET AL: "Synergistic induction of apoptosis by the histone deacetylase inhibitor SAHA and the cyclin-dependent kinase flavopiridol in human leukemia cells" PROCEEDINGS OF THE AMERICAN ASSOCIATION FOR CANCER RESEARCH ANNUAL, vol. 43, March 2002 (2002-03), page 879 XP001156355 93rd Annual Meeting of the American Association for Cancer Research;San Francisco, California, USA; April 06-10, 2002, March, 2002 ISSN: 0197-016X
- YU CHUNRONG ET AL: "Histone deacetylase inhibitors promote STI571-mediated apoptosis in STI571-sensitive and -resistant Bcr/Abl+ human myeloid leukemia cells." CANCER RESEARCH, vol. 63, no. 9, 1 May 2003 (2003-05-01), pages 2118-2126, XP004374567 ISSN: 0008-5472 (ISSN print)
- NIMMANAPALLI RAMADEVI ET AL: "Cotreatment with the histone deacetylase inhibitor suberoylanilide hydroxamic acid (SAHA) enhances imatinib-induced apoptosis of Bcr-Abl-positive human acute leukemia cells." BLOOD, vol. 101, no. 8, 15 April 2003 (2003-04-15), pages 3236-3239, XP002263976 ISSN: 0006-4971
- DATABASE CA [Online] CHEMICAL ABSTRACTS SERVICE, COLUMBUS, OHIO, US; GRISOLANO, JAY L. ET AL: "An activated receptor tyrosine kinase, TEL/PDGF.beta.R, cooperates with AML1/ETO to induce acute myeloid leukemia in mice" retrieved from STN Database accession no. 139:228345 XP002263977 & PROCEEDINGS OF THE NATIONAL ACADEMY OF SCIENCES OF THE UNITED STATES OF AMERICA (2003), 100(16), 9506-9511 ,

## Description

The invention relates to a combination for simultaneous, separate or sequential use which comprises (a) at least one histone deacetylase inhibitor and (b) N-{5-[4-(4-methyl-piperazino-methyl)-benzoylamido]-2-methylphenyl}-4-(3-pyridyl)-2-pyrimidine-amine (designated hereinafter as Compound I) or a pharmaceutically acceptable salt thereof, and optionally at least one pharmaceutically acceptable carrier for simultaneous, separate or sequential use, e.g. in the treatment of leukemia; a pharmaceutical composition comprising such a combination; and the use of the combination of (a) and (b) for the preparation of a medicament for the treatment of leukemia,

Chronic myelogenous leukemia (CML) represents a clonal disorder of a primitive hematopoietic stem cell that results in the progressive accumulation of progenitor cells that are impaired in their capacity to undergo maturation. From a pathophysiologic standpoint, the development of CML represents a consequence of expression of the Bcr/Abl oncogene, which encodes a fusion protein that is found in the cells of 95% of patients with the disease. Constitutive activation of the Bcr/Abl tyrosine kinase confers hematopoietic cells with a survival advantage, contributing to leukemic transformation. In addition to protecting hematopoietic cells from certain noxious environmental stimuli (e.g., growth factor deprivation), expression of the Bcr/Abl kinase renders cells relatively insensitive to apoptosis induced by cytotoxic drugs. Currently, the pathways downstream of Bcl/Abl responsible for apoptosis resistance in CML cells are not known with certainty. However, multiple signaling/survival pathways have been implicated in this phenomenon, including dysregulation of NFk-B, Stat5, MEK/MAP kinase, Bcl-x_{L}, and Akt, among others.

Recently, the treatment of CML has been revolutionized by the introduction of Compound I, a orally active tyrosine kinase inhibitor that inhibits Bcr/Abl, c-Kit, PDGF and other kinases. Compound I interferes with the growth of and induces apoptosis in Bcr/Abl-positive leukemia cells *in vitro*. Significantly, oral administration of Compound I to CML patients results in clinical responses in >90% patients. However, the emergence of Compound I resistance in CML patients initially responsive to this agent, as well as the observation that patients in accelerated phase CML or blast crisis are less likely to respond to Compound I, have prompted the search for additional approaches to the treatment of this disease.

Mechanisms of resistance to Compound I include diminished drug uptake, Bcr/Abl amplification, and mutations in the Bcr/Abl kinase domain, among others. One possible approach to this problem involves the combination of Compound I with other agents that exhibit anti-leukemic activity. In this regard, increased activity against Bcr/Abl⁺ leukemic cells has been described when Compound I was combined with conventional cytotoxic drugs, arsenic trioxide, geldanamycin, or tumor necrosis factor apoptosis-inducing ligand (TRAIL). Most recently, synergistic interactions between Compound I and pharmacologic MEK1/2 inhibitors, e.g. PD184351, U0126 or the cyclin-dependent kinase inhibitor flavopiridol in Bcr/Abl+ cells has been described, including those resistant to Compound I due to increased Bcr/Abl protein expression.

Histone deacetylase inhibitors (HDIs), including trichostatin A, sodium butyrate, suberoylanilide hydroxamic acid (SAHA), depsipeptide, MS-275, and aphicidin, among others, represent a novel class of agents that act by promoting histone acetylation, resulting in relaxation of the chromatin structure. Chromatin relaxation and uncoiling permits the expression of diverse genes, including those involved in the differentiation process, e.g. p21^{CIP1}. In fact, HDIs, e.g. SAHA, sodium butyrate, have been shown to induce maturation in various human leukemia cell lines. Under some circumstances, HDIs induce apoptosis rather than maturation in human leukemia cells, although the factors that determine which response predominates remain obscure. HDIs also induce maturation in certain Bcr/Abl⁺ leukemia cells, e.g. K562, a phenomenon associated with diminished activation of the MAP kinase pathway.

Compound I may modify the differentiation response of Bcr/Abl⁺ cells and it was surprisingly found that combining Compound I with HDIs might promote maturation or otherwise alter leukemic cell survival. To address this issue, interactions between Compound I with clinically relevant HDIs, i.e. sodium butyrate and SAHA, are examined. Co-administration of HDIs with Compound I in several CML cell lines, e.g. K562, LAMA 84, results in disruption of multiple signaling pathways, induction of mitochondrial injury, and a dramatic potentiation of apoptosis. Moreover, this drug combination potently induces cell death in Compound I-resistant Bcr/Abl+ cells displaying increased Bcr/Abl expression. Together, these findings suggest that the strategy of combining Compound I with clinically relevant HDIs warrants consideration in CML and related hematologic malignancies.

Reversible acetylation of histones is a major regulator of gene expression that acts by altering accessibility of transcription factors to DNA. In normal cells, histone deacetylase (HDA) and histone acetyltransferase together control the level of acetylation of histones to maintain a balance. Inhibition of HDA results in the accumulation of hyperacetylated histones, which results in a variety of cellular responses.

Inhibitors of HDA have been studied for their therapeutic effects on cancer cells. For example, butyric acid and its derivatives, including sodium phenylbutyrate, have been reported to induce apoptosis in vitro in human colon carcinoma, leukemia and retinoblastoma cell lines. However, butyric acid and its derivatives are not useful pharmacological agents because they tend to be metabolized rapidly and have a very short half-life *in vivo*. Other inhibitors of HDA that have been widely studied for their anti-cancer activities are trichostatin A and trapoxin. Trichostatin A is an antifungal and antibiotic and is a reversible inhibitor of mammalian HDA. Trapoxin is a cyclic tetrapeptide, which is an irreversible inhibitor of mammalian HDA. Although trichostatin and trapoxin have been studied for their anti-cancer activities, the *in vivo* instability of the compounds makes them less suitable as anti-cancer drugs. There remains a need for an active compound that is suitable for treating tumors, including cancerous tumors, that is highly efficacious and stable.

Surprisingly, it has now been found that the effect, in treating leukemia, of a combination which comprises (a) at least one histone deacetylase inhibitor and (b) Compound I or a pharmaceutically acceptable salt thereof is greater than the effects that can be achieved with either type of combination partner alone, i.e. greater than the effects of a mono-therapy using only one of the combination partners (a) and (b) as defined herein.

The present invention relates to a combination for simultaneous, separate or sequential use, such as a combined preparation or a pharmaceutical fixed combination, which comprises synergistically effective amounts of (a) at least one histone deacetylase inhibitor and (b) Compound I or a pharmaceutically acceptable salt thereof, wherein the active ingredients are present in each case in free form or in the form of a pharmaceutically acceptable salt, and optionally at least one pharmaceutically acceptable carrier.

The present disclosure relates to a method of treating a warm-blooded animal having leukemia, comprising administering to said animal (a) at least one histone deacetylase inhibitor and (b) Compound I in a quantity which is jointly therapeutically effective against leukemia.

The term "leukemia" as used herein includes, but is not limited to, chronic myelogenous leukemia (CML) and acute lymphocyte leukemia (ALL), especially Philadelphia-chromosome positive acute lymphocyte leukemia (Ph+ ALL). Preferably, the variant of leukemia to be treated by the methods disclosed herein is CML as well as Compound I-resistant leukemia, Bcr/Abl+ leukemia resistant to Compound I.

The term "Compound I resistant leukemia" as used herein defines especially a leukemia in which Compound I or a pharmaceutically acceptable salt thereof shows a reduction of its therapeutic effectiveness, it included but is not restricted to leukemia exhibiting resistance to Compound I treatment due to Bcr/Abl gene amplification, increased expression of the Bcr/Abl protein and Abl kinase domain mutation.

The term "treatment" as used herein includes the administration of the combination partners to a warm-blooded animal, preferably a human, in need of such a treatment with the aim to cure the disease or to have an effect on disease regression or on the delay of progression of the disease.

The term "delay of progression" as used herein means that the disease progression is at lest slowed down or hampered by the treatment and that the patient exhibit survival rate that are improved in comparison to patients not being treated or being treated with the monotherapy.

The combination partner (a) Compound I is N-{5-[4-(4-methyl-piperazino-methyl)-benzoylamido]-2-methylphenyl}-4-(3-pyridyl)-2-pyrimidine-amine having the formula I 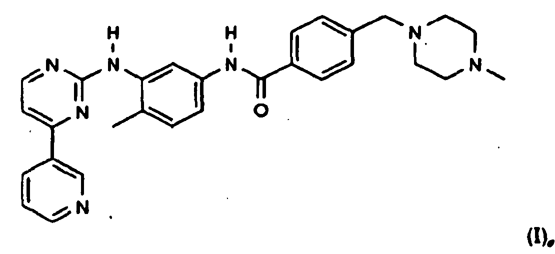 Compound I can be prepared and administered as described in WO 99/03854, especially the monomethanesulfonate salt of N-{5-[4-(4-methyl-piperazino-methyl)-benzoylamido]-2-methylphenyl}-4-(3-pyridyl)-2-pyrimidine-amine can be formulated as described in Examples 4 and 6 of WO 99/03854. Compound I can be administered as marketed under the trademark GLIVEC™ or GLEEVEC™. The term "Compound I" includes all the pharmaceutically acceptable salt thereof and may also be used in form of an hydrate or includes crystal forms, e.g. alpha and beta crystal form, such as described in the European patent application No. 998 473 published on May 10, 2000.

The term "histone deacetylase inhibitors" as used herein includes, but is not limited to sodium butyrate, MS-275 (formerly MS-27-275), suberoylanilide hydroxamic acid (SAHA), aphacidin, depsipeptide, FK228 (formerly FR901228), Trichostatin A and the compounds disclosed in the international patent applications WO 01/38322 (Priority date: 23 Nov.1999) and WO 02/22577 (Priority date: 01 Sept. 2000) filed in the name of NOVARTIS AG, which are hereby incorporated by reference. In particular Compound II of the formula II 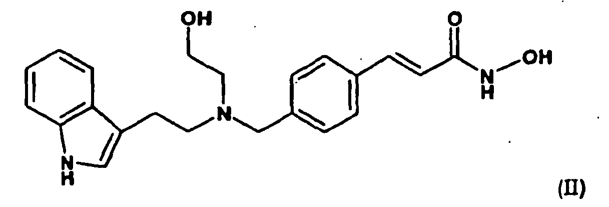 in free form or in the form of a pharmaceutically acceptable salt, preferably in the form of its lactate salt
and

Compound III of the formula III 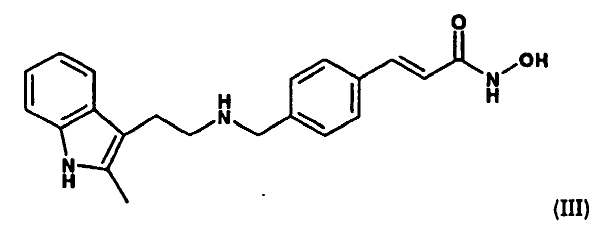 in its free form or in pharmaceutically acceptable salt thereof.

Compound II is specifically disclosed in Example P2 of the international patent application WO 02/22377 published in March 21, 2002, and filed in the name of NOVARTIS AG.

Compound III is specifically disclosed in Example 200 of the international patent application WO 02/22377 published in March 21, 2002, and filed in the name of NOVARTIS AG. Compound III is in free form or in the form of a pharmaceutically acceptable salt.

The structure of the active agents identified by code numbers, generic or trade names may be taken from the actual edition of the standard compendium "The Merck Index" or from databases, e.g. Patents International (e.g. IMS World Publications). The corresponding content thereof is hereby incorporated by reference.

The present invention pertains to a combination, such as a combined preparation or a pharmaceutical composition, which comprises (a) the Compound I or a pharmaceutically acceptable salt thereof, and (b) at least one histone deacetylase inhibitor selected from suberoylanilide hydroxamic acid (SAHA), , Compound II and Compound III, wherein the active ingredients are present in each case in free form or in the form of a pharmaceutically acceptable salt, and optionally at least one pharmaceutically acceptable carrier; for simultaneous, separate or sequential use.

The present invention pertains to a combination, such as a combined preparation or a pharmaceutical composition, which comprises (a) the Compound I or a pharmaceutically acceptable salt thereof and (b) at least one histone deacetylase inhibitor selected from suberoylanilide hydroxamic acid (SAHA). Compound II and Compound III, wherein the active ingredients are present in each case in free form or in the form of a pharmaceutically acceptable salt, and optionally at least one pharmaceutically acceptable carrier; for simultaneous, separate or sequential use.

When the combination partners employed in the COMBINATION OF THE INVENTION are applied in the form as marketed as single drugs, their dosage and mode of administration can take place in accordance with the information provided on the package insert of the respective marketed drug in order to result in the beneficial effect described herein, if not mentioned herein otherwise.

The subject-matter of the final products, the pharmaceutical preparations and the claims of the patent rights cited herein-above is hereby incorporated into the present application by reference. Comprised are likewise the corresponding stereoisomers as well as the corresponding crystal modifications, e.g. solvates and polymorphs, which are disclosed therein. The compounds used as active ingredients in the combinations disclosed herein can be prepared and administered as described in the cited documents, respectively, if not otherwise mentioned herein.

It will be understood that references to the combination partners (a) and (b) are meant to also include the pharmaceutically acceptable salts. If these combination partners (a) and (b) have, for example, at least one basic center, they can form acid addition salts. Corresponding acid addition salts can also be formed having, if desired, an additionally present basic center. The combination partners (a) and (b) having an acid group (for example COOH) can also form salts with bases. The combination partner (a) or (b) or a pharmaceutically acceptable salt thereof may also be used in form of a hydrate or include other solvents used for crystallization.

The term "a combined preparation", as used herein defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b). The ratio of the total amounts of the combination partner (a) to the combination partner (b) to be administered in the combined preparation can be varied, e.g. in order to cope with the needs of a patient sub-population to be treated or the needs of the single patient which different needs can be due to the particular disease, age, sex, body weight, etc. of the patients. Preferably, there is at least one beneficial effect, e.g., a mutual enhancing of the effect of the combination partners (a) and (b), in particular a synergism, e.g. a more than additive effect, additional advantageous effects, less side effects, a combined therapeutical effect in a non-effective dosage of one or both of the combination partners (a) and (b), and very preferably a strong synergism of the combination partners (a) and (b).

In one embodiment of the disclosure leukemia, in particular Compound I-resistant leukemia, is treated with a combination comprising as combination partners (a) Compound I and (b) a histone deacetylase inhibitor selected from the group consisting of sodium butyrate, MS-275 (formerly MS-27-275), suberoylanilide hydroxamic acid (SAHA), aphacidin, depsipeptide, FK228 (formerly FR901228), Trichostatin A, SAHA, Compound II and Compound III. Preferably, the histone deacetylase inhibitor is selected from sodium butyrate, SAHA, Compound II, and Compound III.

A combination which comprises (a) Compound I or a pharmaceutically acceptable salt thereof and (b) a histone deacetylase inhibitor selected from the group consisting of suberoylanilide hydroxamic acid (SAHA), , Compound II and Compound III, in which the active ingredients are present in each case in free form or in the form of a pharmaceutically acceptable salt and optionally at least one pharmaceutically acceptable carrier, will be referred to hereinafter as a COMBINATION OF THE INVENTION.

A combination which comprises (a) Compound I or a pharmaceutically acceptable salt thereof, and (b) a histone deacetylase inhibitor selected from the group consisting of , suberoylanilide hydroxamic acid (SAHA), Compound II and Compound III, in which the active ingredients are present in each case in free form or in the form of a pharmaceutically acceptable salt and optionally at least one pharmaceutically acceptable carrier, will be referred to hereinafter as a COMBINATION OF THE INVENTION.

The COMBINATIONS OF THE INVENTION inhibit the growth of leukemia, e.g. CML, Compound I-resistant leukemia. In one embodiment of the invention, the proliferative disease to be treated with a COMBINATION OF THE INVENTION is leukemia, especially Bcr/Abl+ leukemia and preferably Compound I-resistant leukemia.

All the more surprising is the experimental finding that in vivo the administration of a COMBINATION OF THE INVENTION compared to a monotherapy applying only one of the pharmaceutically active ingredients used in the COMBINATION OF THE INVENTION results not only in a more beneficial, especially synergistic, e.g. anti-proliferative effect, e.g. with regard to the delay of progression of a proliferative disease or with regard to a change in tumor volume, but also in further surprising beneficial effects, e.g. less side-effects and a decreased mortality and morbidity. The COMBINATIONS OF THE INVENTION are suitable in particular in the treatment of proliferative diseases refractory to chemotherapeutics knowns as anti-cancer agents as well as proliferative diseases refractory to Compound I treatment.

A further benefit is that lower doses of the active ingredients of the COMBINATION OF THE INVENTION can be used, for example, that the dosages need not only often be smaller but are also applied less frequently, or can be used in order to diminish the incidence of side-effects like, e.g., diarrhea or nausea observed with one of the combination partners alone. This is in accordance with the desires and requirements of the patients to be treated.

It can be shown by established test models that a COMBINATION OF THE INVENTION results in the beneficial effects described herein before. The person skilled in the pertinent art is fully enabled to select a relevant test model to prove such beneficial effects. The pharmacological activity of a COMBINATION OF THE INVENTION may, for example, be demonstrated in a clinical study or in a test procedure as essentially described hereinafter.

suitable clinical studies are in particular randomized, double-blind, placebo-controlled, parallel studies in cancer patients with late stage disease. Such studies are, in particular, suitable to compare the effects of a monotherapy using the active ingredients and a therapy using a COMBINATION OF THE INVENTION, and to prove in particular the synergism of the active ingredients of the COMBINATIONS OF THE INVENTION. The primary endpoints in such studies can be the effect on pain scores, analgesic use, performance status, Quality of Life scores or time to progression of the disease. The evaluation of tumors by in regular time periods, e.g. every 4, 6 or 8 weeks, is a suitable approach to determine the effect of the COMBINATION OF THE INVENTION.

It is one objective of this invention to provide a pharmaceutical composition comprising a quantity, which is jointly therapeutically effective against a proliferative disease comprising the COMBINATION OF THE INVENTION. In this composition, the combination partners (a) and (b) can be administered together, one after the other or separately in one combined unit dosage form or in two separate unit dosage forms. The unit dosage form may also be a fixed combination.

The pharmaceutical compositions according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application. In one embodiment of the disclosure one or more of the active ingredients are administered intravenously.

The novel pharmaceutical composition contain, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partners of the COMBINATION OF THE INVENTION may be administered simultaneously or sequentially and in any order, and the components may be administered separately or as a fixed combination. For example, the method of delay of progression or treatment of a proliferative disease according to the invention may comprise (i) administration of the first combination partner in free or pharmaceutically acceptable salt form and (ii) administration of the second combination partner in free or pharmaceutically acceptable salt form, simultaneously or sequentially in any order, in jointly therapeutically effective amounts, preferably in synergistically effective amounts, e.g. in daily dosages corresponding to the amounts described herein. The individual combination partners of the COMBINATION OF THE INVENTION can be administered separately at different times during the course of therapy or concurrently in divided or single combination forms. Furthermore, the term administering also encompasses the use of a pro-drug of a combination partner that convert *in vivo* to the combination partner as such. The instant disclosure is therefore to be understood as embracing all such regimes of simultaneous or alternating treatment and the term "administering" is to be interpreted accordingly.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites. This involves a consideration of the distribution, equilibrium, and elimination of the active ingredients. 119.5 mg of Compound I monomethanesulfonate correspond to 100 mg of COMPOUND I (free base) as active substance. Depending on species, age, individual condition, mode of administration, and the clinical picture in question, effective doses of Compound I, for example daily doses corresponding to about 50 to 1000 mg, e.g. 50 to 800 mg of the active substance, preferably 50 to 600 mg, e.g. 50 to 400 mg, are administered to warm-blooded animals of about 70 kg bodyweight. For adult patients with leukemia, a starting dose of 400 mg daily may be recommended. For patients with an inadequate response after an assessment of response to therapy with 400 mg daily, dose escalation can be safely considered and patients may be treated as long as they benefit from treatment and in the absence of limiting toxicities.

The disclosure relates also to a method for administering to a human subject suffering from a leukemia COMBINATION OF THE INVENTION wherein a pharmaceutically effective amount of Compound I or a pharmaceutically acceptable salt thereof is administered to said human subject daily for a period exceeding 3 months. The invention relates especially to such method wherein a daily dose of 50 to 800 mg of the active substance, especially 50 to 600 mg, e.g. 50 to 400 mg, is administered.

When the combination partners employed in the COMBINATION OF THE INVENTION are applied in the form as marketed as single drugs, their dosage and mode of administration can take place in accordance with the information provided on the packet leaflet of the respective marketed drug in order to result in the beneficial effect described herein, if not mentioned herein otherwise.

The COMBINATION OF THE INVENTION can be a combined preparation or a pharmaceutical composition.

Moreover, the disclosure relates to a method of treating a warm-blooded animal having a leukemia comprising administering to the animal a COMBINATION OF THE INVENTION in a quantity which is jointly therapeutically effective against a proliferative disease and in which the combination partners can also be present in the form of their pharmaceutically acceptable salts. In one embodiment of the invention, in such method the COMBINATION OF THE INVENTION is co-administered with an anti-diarrheal agent. Furthermore, the treatment can comprise surgery, radiotherapy, cryotherapy and immunotherapy.

The disclosure also relates to a method of inhibiting the formation of metastases in a warm-blooded animal having a leukemia which comprises administering to the patient a pharmaceutically effective amount of the COMBINATION OF THE INVENTION in a quantity which is jointly therapeutically effective against said leukemia and in which the compounds can also be present in the form of their pharmaceutically acceptable salts.

The present invention pertains to the use of a COMBINATION OF THE INVENTION for the treatment of leukemia, e.g. Compound I-resistant leukemia and for the preparation of a medicament for the treatment of a leukemia.

Additionally, the disclosure pertains to the use of Compound I or a pharmaceutically acceptable salt thereof in combination with for the preparation of a medicament for the treatment of aleukemia.

Moreover, the disclosure describes a commercial package comprising as active ingredients COMBINATION OF THE INVENTION, together with instructions for simultaneous, separate or sequential use thereof in the treatment of leukemia, e.g. CML, Compound I resistant leukemia.

The present disclosure relates to a method of treating a warm-blooded animal having a proliferative disease, preferably BCR/ABL+ human myeloid leukemia, comprising administering to said animal a combination which comprises (a) Compound I, especially in the form of its monomethanesulfonate salt, and (b) a histone deacetylase inhibitor selected from SAHA, sodium butyrate, Compound II and Compound III, in a quantity which is jointly therapeutically effective against said proliferative disease and in which the compounds can also be present in the form of their pharmaceutically acceptable salts.

The present invention preferably relates to the use of a combination, which comprises (a) Compound I or a pharmaceutically acceptable salt thereof, and (b) a histone deacetylase inhibitor selected from the group consisting of SAHA, Compound II and Compound III, as described herein, for the preparation of a medicament for the treatment of a proliferative disease, preferably BCR/ABL+ human myeloid leukemia, most preferably a Compound I-resistant BCR/ABL⁺ human myeloid leukemia.

### EXAMPLE 1:

### MATERIALS AND METHODS

*Cells:* K562, HL60, Jurkat and U937 human leukemia cells are purchased from American Type Culture Collection, Rockville, MD. LAMA 84 cells are purchased from the German Collection of Microorganisms and Cell Cultures (Braunschweig, Germany). All cells are cultured in RPMI 1640 supplemented with sodium pyruvate, MEM essential vitamins, L-glutamate, penicillin, streptomycin, and 10% heat-inactivated FCS (Hyclone, Logan, UT). They are maintained in a 37°C, 5% CO₂, fully humidified incubator, passed twice weekly, and prepared for experimental procedures when in log-phase growth (cell density ≤4 x 10⁵ cells/ml).

Multidrug-resistant K562R cells are derived from the parental line by subculturing in progressively higher concentrations of doxorubicin as described previously (Yanovich et al., Cancer Res 1989, 49:4499-4503. They are cultured in the absence of doxorubicin before all of the experimental procedures. In addition, Compound I-resistant LAMA 84 cells, designated LAMA 84-R, are generated by subculturing LAM 84 cells in progressively higher concentrations of Compound I. These cells are maintained under selection pressure in medium containing 0.5 µM of Compound I. I.C.₅₀ Compound I values for LAMA-S-571 and LAMA-R-571 are 0.3 vs 1.8 µM respectively. For studies involving the LAMA 84-R line, cells are washed free of drug and resuspended in drug-free medium 48 h before experimentation.

*Reagents:* Compound I is prepared as a 10 mM stock solution in sterile DMSO (Sigma Chemical Co., St. Louis, MO). Sodium butyrate and SAHA are supplied from Calbiochem, San Diego, CA; BOC-fmk and IETD-fmk are purchased from Enzyme Products, Ltd., Livermore, CA, and formulated in sterile DMSO before use.

*Experimental Format:* Logarithmically growing cells are placed in sterile plasticT-flasks (Corning, Corning, NY) to which are added the designated drugs and the flasks replaced in the incubator for intervals. At the end of the incubation period, cells are transferred to sterile centrifuge tubes, pelleted by centrifugation at 400xg for 10 min at room temperature, and prepared for analysis as described below.

*Assessment of Apoptosis:* After drug exposures, cytocentrifuge preparations are stained with Wright-Giemsa and viewed by light microscopy to evaluate the extent of apoptosis (i.e., cell shrinkage, nuclear condensation, formation of apoptotic bodies, etc.) as described previously (Yu et al., Nat. Rev. Cancer 1:294-202) . For these studies, the percentage of apoptotic cells is determined by evaluating ≥500 cells/condition in triplicate. To confirm the results of morphological analysis, Annexin V/PI staining is used. Annexin V/PI (BD PharMingen, San Diego, CA) analysis of cell death is carried out as per the manufacturer's instructions. In studies involving TNF and TNF soluble receptor, both compound are combined and maintained at room temperature 20 min prior to use. For these experiments 1-2x10⁵ cells per condition are harvested. Analysis is carried out using a Becton-Dickinson FACScan cytofluorometer (Mansfield, MA). To further confirm the morphology results, TUNEL staining is used. For TUNEL staining, cytocentrifuge preparations are obtained and fixed with 4% formaldehyde. The slides are treated with acetic acid/ethanol (1:2), stained with terminal transferase reaction mixture containing 1 x terminal transferase reaction buffer (0.25 units/µl terminal transferase, 2.5 mM CoCl₂, and 2 pmol fluorescein-12-dUTP; Boehringer Mannheim, Indianapolis, IN), and visualized using fluorescence microscopy.

*Determination of MMP*(ΔΨₘ): MMP is monitored using DiOC6 [36]. For each condition, 4 x 10⁵ cells are incubated for 15 min at 37°C in 1 ml of 40 nM DiOC6 (Calbiochem) and subsequently analyzed using a Becton Dickinson FACScan cytofluorometer with excitation and emission settings of 488 and 525 nm, respectively. Control experiments documenting the loss of ΔΨₘ are performed by exposing cells to 5 µM of carbamoyl cyanide m-chlorophenylhydrazone (Sigma Chemical Co.; 15 min, 37°C), an uncoupling agent that abolishes the MMP.

*Preparation of S-100 Fractions and Assessment of Cytochrome C Release:* U937 cells are harvested after drug treatment as described previously (Yu et al., 2001, BBRC 286:1011-18) by centrifugation at 600xg for 10 min at 4°C and washed in PBS. Cells (4x10⁶) are lysed by incubating for 3 min in 100 µl of lysis buffer containing 75 mM NaCl, 8 mM Na₂HPO₄, 1 mM NaH₂PO₄, 1 mM EDTA, and 350 µg/ml digitonin. The lysates are centrifuged at 12,000*xg* for 5 min, and the supernatant is collected and added to an equal volume of 2xLAEMMLI buffer. The protein samples are quantified and separated by 15% SDS-PAGE.

*Immunoblot Analysis:* Immunoblotting is performed as described previously (Yu et al., Nat. Rev. Cancer 1:294-202). In brief, after drug treatment cells are pelleted by centrifugation, and lysed immediately in Laemmli buffer [1X = 30 mM Tris-base (pH 6.8), 2% SDS, 2.88 mM ßmercaptoethanol, and 10% glycerol], and briefly sonicated. Homogenates are quantified using Coomassie protein assay reagent (Pierce, Rockford, IL). Equal amounts of protein (20 µg) are boiled for 10 min, separated by SDS-PAGE (5% stacker and 10% resolving), and electroblotted to nitrocellulose membrane. After blocking in TBS-T (0.05%) and 5% milk for 1 h at 22°C, the blots are incubated in fresh blocking solution with an appropriate dilution of primary antibody for 4 h at 22°C. The source of antibodies are as follows: Bcl-x_{L}, rabbit polyclonal, Santa Cruz Biotechnology; XIAP, rabbit polyclonal, R&D Systems, Minneapolis, MN; Mcl-1, mouse monoclonal Pharmingen, San Diego, CA; cyclin D1, mouse monoclonal; p21, mouse monoclonal, Pharmingen; ERK 1/2, rabbit polyclonal, Cell Signaling Technology, Beverly, MA; phospho-ERK 1/2 (thr202/tyr204), rabbit polyclonal, Cell Signaling Technology; JNK, rabbit polyclonal, Santa Cruz Biotechnology; phospho-JNK, mouse monoclonal, Santa Cruz Biotechnology; phospho-p38 MAPK, rabbit polyclonal, Cell Signaling Technology; phospho-p70S6K(421/424), rabbit polyclonal, Cell Signaling Technology; phosphor-STAT5, Cell Signaling Technology; pRB, mouse monoclonal, Pharmingen; under-phospho-RB, mouse monoclonal, PharMingen; caspase 3, mouse monoclonal, Transduction Laboratories, Lexington, KY; PARP (C-2-10), mouse monoclonal, BioMol Research Laboratories, Plymouth, MA; cytochrome c, mouse monoclonal, Santa Cruz Biotechnology; AIF, mouse monoclonal, Santa Cruz Biotechnology; Smac, rabbit polyclonal, Upstate Biotechnology, Lake Placid, NY; caspase 8, rabbit polyclonal, Pharmingen; and α-tubulin, Calbiochem. Blots are washed 3x15 min in TBS-T and then incubated with a 1:2000 dilution of horseradish peroxidase-conjugated secondary antibody (Bio-Rad Laboratories, Hercules, CA) for 1 h at 22°C. Blots are again washed 3x15 min in TBS-T and then developed by enhanced chemiluminescence (Pierce, Rockford, IL).

### Differentiation Studies

Analysis of erythroid maturation of K562 cells is performed by monitoring the production of hemoglobin as previously described (Yang et al., J. Biol.Chem. 2001, 276:25742-52). *Clonogenic Survival:* Effects of drug treatment on the clonogenic survival of leukemia cells is determined using a previously described clonogenic assay (Yu et al., Mol. Pharmacol. 2001, 60: 143-54).

*Transient transfections:* Plasmids encoding enhanced green fluorescence protein under the transcriptional control of the human cytomegalovirus (CMV) immediate-early promoter (pEGFP-C2), and HA-tagged activated MEK1 (S218D/S222D in pUSEEamp) are obtained from Clontech Laboratories (Palo Alto, CA) and Upstate Biotechnology (Lake Placid, NY), respectively. A 1285-bp fragment containing the *MEK1* cDNA was obtained by Apa I and partial EcoR I digestion and inserted in-frame into the (C-terminal) multiple cloning site of pEGFP-C2. The entire *MEK1* cDNA in the fusion construct is sequenced and the reading frame is confirmed. Log-phase K562 cells is transfected in electoporation hypoosmolar buffer (Eppendorf ) using a BTX electromanipulator 600. 20 µg DNA and 2.0x10⁷ cells are used for each condition. After 12 hour of incubation, 20% to 30% of the cells displayed green fluorescence. The brightest 10% to 20% of the total cell population is isolated by fluorescence-activated cell sorting (FACS) using a Cytomation MoFLO cell sorter. The cells are then exposed to drugs as indicated, and examined for morphologic evidence of apoptosis as described above.

***Statistical Analysis*:** The significance of differences between experimental conditions is determined using the two-tailed Student t test. Analysis of synergism and antagonism is performed using Median Dose Effect analysis (Chou and Talalay, 1984, Adv.Enz. Regul. 22:27-55) in conjunction with a commercially available software program (Caleysyn; Biosoft;.. Ferguson, MO) as previously described Yu et al., 2002, Cancer Res. 62:188-189).

### RESULTS

To characterize interactions between Compound I and SAHA in K562 cells, dose response studies are performed. Exposure of cells for 24 hr to Compound I concentrations as high as 300 nM neglibly induced apoptosis, while 2.0 µM SAHA administered alone is also minimally-toxic. However, when cells are exposed to SAHA in combination with 100 nM Compound I, a clear increase in apoptosis is observed (i.e., ~ 20%), and for Compound I concentrations of 250 nM, the large majority of cells (i.e., ~75%) are apoptotic (Table 1A).

**Table 1A: K562 cells are exposed for to 2.0 µM SAHA in conjunction with the indicated concentration of Compound I after which apoptosis is monitored by morphological analysis of Wright Giemsa-stained specimens as described in Methods.**

| Compound I (nM) | SAHA (µM) | |
|---|---|---|
| | 0 | 2 |
| 0 | 0.6±0.3 | 3.2±1.1 |
| 100 | 1.1±0.5 | 20.4±3.2 |
| 150 | 1.3±0.6 | 34.5±3.8 |
| 200 | 1.6±0.6 | 44.6±4.1 |
| 250 | 2.8±1.2 | 65.3±4.3 |
| 300 | 6.3±2.1 | 71.2±4.8 |

Similarly, when cells are exposed for 24 hr to 250 nM Compound I in combination with increasing concentrations of SAHA, a sharp increase in apoptosis is noted at 1.0 µM SAHA, and at SAHA concentrations of 1.5 µM, the majority of cells are apoptotic (Table 1B).

**Table 1B. Cells are exposed for 24 hr to 250 nM Compound I in conjunction with the designated concentration of SAHA, after which apoptosis is assessed as above. For A and B, values represent the means ± S.D. for three separate experiments.**

| SAHA (µM) | Compound I (nM) | |
|---|---|---|
| | 0 | 250 nM |
| 0 | 0.6±0.2 | 2.7±1.2 |
| 1 | 0.8±0.4 | 21.4±3.2 |
| 1.5 | 1.6±0.7 | 52.3±4.3 |
| 2.0 | 2.7±0.7 | 64.5±4.5 |
| 2.5 | 14.5±1.3 | 68.9±4.7 |
| 3.0 | 28.4±3.2 | 77.8±5.1 |

Median Dose Effect analysis of apoptosis induction over a range of and SAHA concentrations yielded Combination Index (CI) values lower than 1.0, corresponding to a synergistic interaction (Table 1 C).

**Table 1C. K562 cells are exposed to varying concentrations of SAHA and Compound I at a fixed ratio (10:1) for 24 hr after which Combination Index (CI) values for apoptosis are determined in relation to the Fraction Affected (FA) using Median Dose Effect analysis. CI values <1.0 correspond to a synergistic interaction. Two additional studies yield similar results.**

| SAHA (mM) | Compound I (mM) | Combination Index (CI) | Fractional Effect |
|---|---|---|---|
| 1.0 | 125 | 0.787 | 0.184 |
| 1.5 | 187.5 | 0.762 | 0.406 |
| 2.0 | 250 | 0.792 | 0.623 |
| 2.5 | 312.5 | 0.834 | 0.727 |
| 3.0 | 375 | 0.794 | 0.893 |

There is a striking increase in apoptosis in K562 cells exposed to 250 nM Compound I + 2.0 µM SAHA for 24 hr as compared to K562 cells exposed to drug-free medium ; SAHA 2.0 µM (24 hr); Compound I 250 nM (24 hr) shown by the photomicrographs of TUNEL-stained cells (not shown).

Time course studies of K562 cells exposed to 250 nM Compound I ± 2.0 µM SAHA are performed. Whereas each of these agents administered individually over 48 hr minimally induced apoptosis, combined treatment resulted in an increase in apoptosis that is first observed at 12 hr, and which reached near-maximal levels by 24 hr. After 48 hr of combined treatment, over 90% of cells are apoptotic (Table 2A).

**Table 2A: K562 cells are exposed to 2.0 µM SAHA ± 250 nM Compound I for the indicated intervals after which the percentage of apoptotic cells is determined as described in Methods.**

| Hours | Control | SAHA | Compound I | SAHA+Compound I |
|---|---|---|---|---|
| 0 | 0.6±0.2 | 0.6±0.2 | 0.6±0.2 | 0.6±0.3 |
| 6 | 0.6±0.3 | 0.9±0.2 | 1.1±0.5 | 6.7±0.8 |
| 12 | 0.6±0.2 | 1.7±0.5 | 2.1±0.8 | 13.7±2.1 |
| 18 | 0.6±0.2 | 2.3±0.8 | 3.3±1.0 | 33.4±3.8 |
| 24 | 0.6±0.3 | 3.5±1.1 | 3.5±1.2 | 65.5±4.8 |
| 48 | 0.6±0.3 | 14.8±2.1 | 21.8±3.4 | 86.3±5.9 |

Similar results are observed when loss of mitochondrial membrane potential (ΔΨΘₘ) is monitored, although Compound I by itself is somewhat more toxic in this regard after 48 hr of exposure (Table 2B).

**Table 2B: K562 cells are exposed to 2.0 µM SAHA ± 250 nM Compound I for the indicated intervals after which the percentage of cells displaying loss of mitochondrial membrane potential (ΔΨₘ) is determined as described in Methods.**

| Hours | Control | SAHA | Compound I | SAHA+Compound I |
|---|---|---|---|---|
| 0 | 5.3±1.8 | 5.4±1.6 | 5.7±1.8 | 5.6±1.7 |
| 6 | 5.8±1.7 | 8.1±1.9 | 8.8±1.9 | 16.5±2.1 |
| 12 | 6.1±1.8 | 10.4±2.1 | 11.2±2.1 | 24.6±3.4 |
| 18 | 5.2±1.6 | 12.3±2.8 | 11.9±2.3 | 45.8±3.8 |
| 24 | 6.4±1.8 | 16.4±2.9 | 17.8±3.4 | 65.8±4.5 |
| 48 | 5.9±1.7 | 20.5±2.8 | 41.3±4.3 | 87.2±5.8 |

Together, these findings indicate that combined treatment with Compound I and the HDI SAHA results in early induction of mitochondrial injury and apoptosis in Bcr/Abl+ K562 cells.

To determine whether potentiation of apoptosis in K562 cells treated with Compound I in conjunction with an HDI would be associated with loss of leukemic cell self-renewal capacity, clonogenic assays are performed. While a 24-hr exposure to 250 nM Compound I or 2.0 µM SAHA individually substantially reduced clonogenicity (i.e. to~25% of control values), combined treatment resulted in greater than a 2-log reduction in colony formation (Table 2C).

**Table 2C: Cells are treated with 2.0 µM SAHA ± 250 nM Compound I for 24 hr, washed free of drugs, and plated in soft agar as described in Methods. At the end of a 12-day incubation period, colonies are scored and survival expressed as a percentage relative to untreated controls.**

| | Clonogenic survival (% control) |
|---|---|
| SAHA | 21.8±3.6 |
| Compound I | 22.4±4.5 |
| SAHA+Compound I | 0.42±0.2 |

Because both Compound I and HDIs such as butyrate have been shown to induce maturation in Bcr/Abl⁺ cells, an attempt is made to determine if combined exposure to such agents would result in an increase in K562 cell differentiation. To this end, hemoglobin (Hgb) production is monitored in K562 cells treated with SAHA ± Compound I. K562 cells are exposed to 2.0 µM SAHA ± 250 nM Compound I for the indicated intervals after which differentiation was monitored by quantifying intracellular levels of Hgb as described in Methods. In each case, values represent the means ± S.D. for three separate experiments. Following a 24-hr exposure, SAHA-treated cells displayed a marked (i.e. ~50%) increase in Hgb production, while Compound I is less effective in this regard. However, cells treated with both agents do not exhibit an increase in Hgb levels. After 48 hr, both SAHA- and Compound I-treated cells exhibit substantial increases in Hgb production. However, levels of Hgb in cells exposed to both agents, which are largely apoptotic at this time, are lower than controls(data not shown). These findings indicate that co-treatment of Bcr/Abl+ K562 cells with Compound I and SAHA does not promote differentiation, but instead suggests that the extensive apoptosis that occurs under these conditions instead prevents this process.

The effects of combined exposure of K562 cells to SAHA and Compound I for 24 hr are then examined in relation to mitochondrial injury, caspase activation, and expression of apoptotic regulatory proteins. K562 cells are exposed to 2.0 µM SAHA ± 250 nM Compound I for 24 hr after which Western analysis is employed to assess release of AIF, Smac/DIABLO, and cytochrome C into S-100 cytosolic fractions, and total cellular extracts are monitored for expression of cleaved caspase 9, caspase-3, caspase-8, PARP, and Bcr/Abl. Each lane contained 25 µg of protein; blots are stripped and re-probed for tubulin to ensure equivalent loading and transfer. Two additional studies yielded equivalent results. Whereas the effects of Compound I (250 nM) or SAHA (2.0 µM) individually are minimal, combined exposure of cells to these agents resulted in a striking increase in release of cytochrome c, AIF, and Smac/DIABLO into the cytosolic, S-100 cell fraction. These events are accompanied by a marked increase in caspase-9 cleavage, and degradation of caspase-3, caspase-8, and PARP. Interestingly, whereas individual treatment has little effect, combined exposure to Compound I and SAHA resulted in a marked decline in levels of the Bcr/Abl protein (data not shown). Thus, treatment of Bcr/Abi+ cells with a sub-toxic concentration of Compound I in conjunction with the HDI SAHA resulted in a marked increase in release of pro-apoptotic mitochondrial proteins, activation of the caspase cascade, and reduced expression of Bcr/Abl.

Interactions between SAHA and Compound I are then examined in relation to effects on various signaling, cell cycle, and apoptotic regulatory proteins in K562 cells. Interestingly, exposure to SAHA alone (16 hr) result in a clear reduction in expression of Raf, whereas Compound I has little effect. K562 cells are exposed to 2.0 µM SAHA ± 250 nM Compound I for 16 hr after which Western analysis is employed to assess expression of Raf, phosphor-MEK1/2 and -ERK1/2, total ERK1/2, phosphor-70^{S6K} (ERK-phosphorylated sites; 421/424); phospho-JNK, phosphor-p38 MAPK, phosphor-STAT 5, phospho-Akt (423), and total Akt. K562 cells are treated as above, and monitored for expression of Bel-X_{L}, Mcl-1, and XIAP. Following treatment with Compound I ± SAHA as above, expression of p21CIP1, under-phosphorylated pRb, total pRb, and cyclin D₁ are examined by Western analysis. CF = cleavage fragment. Each lane contained 25 µg of protein; blots are stripped and re-probed for tubulin to ensure equivalent loading and transfer. Two additional studies yield equivalent results ( data not shown). Co-administration of Compound I and SAHA resulted in a further diminution in Raf expression. Roughly parallel changes in levels of phospho-MEK1/2 and phospho-ERK1/2 are observed. Combined treatment with Compound I and SAHA also resulted in a marked decrease in phosphorylation of p70^{S6K} on ERK-associated sites (421/424), as well as markedly diminished expression of phospho-STAT5, a target of Bcr/Abl. No changes in the expression of total Akt are noted, although a modest decline in phosphorylated (activated) Akt is observed in cells exposed to both SAHA and Compound I. In addition, while Compound I and SAHA individually fail to modify expression of phospho- JNK, combined treatment resulted in a very dramatic increase in JNK activation. A slight increase in phosphorylation of p38 MAPK is observed in SAHA-treated cells, but this does not change with addition of Compound I. Combined treatment with Compound I and SAHA do not alter expression of the anti-apoptotic proteins Bcl-x_{L} or XIAP. However, Compound I treatment alone induces a small decrease in expression of the anti-apototic protein Mcl-1, as previously reported, while addition of SAHA, which by itself exerted minimal effects, resulted in a further diminution in Mcl-1 expression (data not shown).

Interactions between SAHA and Compound I are then examined in relation to expression of several cell cycle regulatory proteins. Treatment with SAHA resulted in a robust induction of p21^{CIP1}, similar to effects noted in Bcr/Abl⁻ leukemia cells. Unexpectedly, co-exposure to Compound I substantially diminished induction of p21^{CIP1} by SAHA. Despite this action, combined exposure of cells to SAHA and Compound I resulted in a modest increase in expression of under-phosphorylated pRb accompanied by cleavage of both total and under-phosphorylated protein. Lastly, K562 cells treated with both Compound I and SAHA displayed a clear reduction in levels of cyclin D₁, a phenomenon previously linked to induction of apoptosis. Collectively, these findings indicate that co-exposure of K562 cells to Compound I and SAHA results in perturbations in the expression of multiple signaling, cell cycle, and apoptotic regulatory proteins, including down-regulation of Raf, diminished activation of MEK1/2, ERK1/2, and p70^{S6K}, a striking activation of JNK, reduced expression of Bcr/Abl, Mcl-1, p21^{CIP1}, and cyclin D₁, and dephosphosphorylation/cleavage of pRb (data not shown).

To assess the role of caspases in these events, K562 cells are treated for 20 hr with Compound I + SAHA in the presence or absence of the pan-caspase inhibitor BOC-fmk or the caspase 8 inhibitor IETD-fmk (Table 3).

**Table 3; K562 cells are exposed to 2.0 µM SAHA + 250 nM Compound I for 24 hr in the presence or absence 25 µM BOC-fmk or IETD-fmk, after which apoptosis is monitored as above. Values represent the means ± S.D. for three separate experiments.**

| | Apoptosis (%) |
|---|---|
| Control | 0.6±0.2 |
| SAHA+Compound I | 50.1±3.9 |
| BOC+SAHA+Compound I | 9.8±2.4 |
| IETD+SAHA+Compound I | 41.5±3.2 |

Cells are treated with SAHA + Compound I ± BOC-fmk, after which release of cytochrome c or Smac/DIABLO into the S-100 cytosolic fraction is assessed as above (not shown). Cells are treated with SAHA + Compound I ± BOC-fmk, after which Western analysis is used to assess expression of procaspase-3, Bcr/Abl, pRb, under phosphorylated pRb, Raf-1, Mcl-1, p21^{CIP1}, and cyclin D₁. Each lane contained 25 µg of protein; blots are stripped and re-probed for tubulin to ensure equivalent loading and transfer. Two additional studies yield equivalent results (data not shown). BOC-fmk markedly inhibited apoptosis whereas IETD-fmk is minimally effective, suggesting a relatively minor role for the extrinsic pathway in Compound I/SAHA-mediated lethality in these cells. However, whereas BOC-fmk is ineffective in blocking cytochrome c release into the cytosol in cells exposed to Compound I + SAHA, it largely blocks Smac/DIABLO release, indicating the latter represents a secondary, caspase-dependent event. As anticipated, BOC-fmk attenuated cleavage of procaspase-3 and both total and under-phosphorylated pRb. It also partially reverses the down-regulation of Bcr/Abl expression in Compound I/SAHA-treated cells, suggesting a caspase-dependent component of this phenomenon. In contrast, BOC-fmk has little effect on down regulation of Raf, Mcl-1, p21^{CIP1}, or cyclin D₁, indicating that these events are largely independent of caspase activation. In separate studies, co-administration of BOC-fmk had no effect on the actions of SAHA administered alone (data not shown).

To determine whether synergism between Compound I and SAHA could be extended to include other Bcr/Abl+ cells, parallel studies are conducted in LAMA 84 cells (Table 4).

**Table 4: LAMA 84 cells are exposed to 200 nM Compound I ± 1.0 µM SAHA for 24 hr, after which the percentage of annexin V/PI+ cells (shown as gated figures) is determined by flow cytometry as described in Methods. Two additional studies yield equivalent results.**

| | % Annexin V/PI |
|---|---|
| Control | 08.2±1.8 |
| SAHA 1mM | 15.2±2.4 |
| Compound I 200nM | 13.4±2.3 |
| SAHA+Compound I | 70.8±4.8 |

Exposure of LAMA 84 cells to 1.0 µM SAHA or 200 nM Compound I alone for 24 hr exerted minimal effects on cell death. However, when the agents are combined, the large majority of cells (i.e., 70%) became apoptotic, reflected by annexin positivity. In contrast, no evidence of synergism is observed when SAHA was combined with several Bcr/Abl- leukemic cell lines, including U937, HL-60, NB4, and Jurkat (Table 5).

**Table 5: Bcr/Abl+ K562 cells, and several Bcr/Abl- leukemia cell lines, including U937 monocytic leukemia, Jurkat lymphoblastic leukemia, and NB4 and HL-60 promyelocytic leukemia cells are exposed to SAHA±Compound I for 24 hr, after which the percentage of apoptotic cells is determined by examining Wright Giemsa-stained cytospin slides as described in Methods. Concentrations for the individual cell lines are as follows: K562: SAHA 2.5 µM; Compound I 250 nM; U937: SAHA 1.5 µM; Compound I 250 nM; Jurkat: SAHA 0.75 µM; Compound I 250 nM; NB4: SAHA 1.5 µM; Compound I 250 nM; HL-60: SAHA 1.0 µM; Compound I 250 nM. In each case, values represent the means ± S.D. for three separate experiments.**

| | % of apoptotic cells | | | |
|---|---|---|---|---|
| Cell line | control | SAHA | Compound I | Compound I+SAHA |
| K562 | 0.6±0.3 | 6.6±3.8 | 8.3±3.2 | 74.3±3.3 |
| U937 | 0.8±0.4 | 5.4±1.9 | 0.9±08 | 6.2±3.0 |
| Jurkat | 0.8±0.4 | 6.3±3.2 | 1.1±0.6 | 7.3±2.0 |
| NB4 | 1.2±0.5 | 5.6±2.3 | 1.8±1.3 | 8.2±2.4 |
| HL60 | 2.1±0.6 | 6.4±1.9 | 3.1±1.3 | 11.5±3.5 |

These findings indicate that synergistic interactions between SAHA and Compound I are restricted to human leukemic cells expressing the Bcr/Abl protein.

Western analysis revealed that combined exposure of LAMA 84 cells to Compound I + SAHA results in a marked increase in cytosolic release of cytochrome c, and a corresponding activation of caspases-9, -3, and -8 (data not shown). Consistent with results obtained in K562 cells, treatment of LAMA 84 cells with the combination of Compound I and SAHA results in down-regulation of Raf, p21^{CIP1}, cyclin D₁, Mcl-1, phospho-STAT5, enhanced under-phosphorylation and cleavage of pRb, and a dramatic increase in JNK phosphorylation (data not shown).

Attempts are then made to establish whether such interactions could be extended to include HDIs other than SAHA. LAMA 84 cells are exposed to 200 nM Compound I ± 1.0 µM SAHA for 24 hr, after which Western analysis is employed to monitor cytochrome c release into the cytosolic S-100 fraction, or expression of cleaved procaspase-9, cleaved procaspase-3, or procaspase-8 in total cell extracts (data not shown). CF = cleavage fragment. LAMA cells are treated as above, after which total cellular extracts are monitored for expression of Raf 1, phospho-JNK, p21^{CIP1}, cyclin D₁, Mcl-1, and phospho-STAT5. Each lane contained 25 µg of protein; blots are stripped and re-probed for tubulin to ensure equivalent loading and transfer. Two additional studies yield equivalent results (data not shown).

To this end, K562 and LAMA 84 cells are exposed for 24 hr to the indicated concentrations of Compound I in the presence or absence of sodium butyrate (SB; 1 or 2 mM), after which the extent of apoptosis is assessed the percentage of apoptotic cells is determined by examining cytospin slides as described in Methods. Values represent the means ± S.D. for three separate experiments (Table 6), co-administration of Compound I with SB resulted in a marked increase in apoptosis in both cell lines.

| | Compound I+SB % of apoptotic cells | | | |
|---|---|---|---|---|
| | control | SB | Compound I | SB+Compound I |
| K562 | 0.6±0.3 | 5.1±2.2 | 10.3±3.6 | 61.2±6.2 |
| LAMA 84 | 1.8±0.5 | 6.4±2.8 | 9.3±3.8 | 55.1±5.4 |

Analogous to results obtained with SAHA, enhanced lethality is associated with increased release of cytochrome c into the cytosol, and activation of procaspases-3 and -9, down-regulation of Raf, p21^{CIP1}, Mcl-1, cyclin D₁, and a marked activation of JNK (data not shown).

Co-administration of Compound I with either MEK1/2 inhibitors or with the cyclin-dependent kinase inhibitors flavopiridol results in enhanced lethality in Compound I-resistant K562 cells exhibiting increased expression of Bcr/Abl. Parallel studies involving the Compound I/HDI regimen are therefore carried out in K562R cells, derived from a multi-drug resistant cell line, as well as in Compound I-resistant LAMA 84 cells (LAMA 84-R), which are generated by culturing cells in progressively higher concentrations of Compound I (Table 7).

**Table 7: Compound I-resistant K562 cells (K562R) and Compound I-resistant LAMA 84 cells (LAMA 84R) are exposed to the indicated concentrations of Compound I and SAHA for 24 hr, after which the percentage of apoptotic cells is determined by examining Wright Giemsa-stained cytospin slides as described in Methods. Insets contain Western blots assaying Bcr/Abl protein levels (along with tubulin controls) in resistant and sensitive cells. Each lane contained 25 µg of protein. Values represent the means ± S.D. for three separate experiments.**

| | Compound I+SAHA % of apoptotic cells | | | |
|---|---|---|---|---|
| | control | SAHA | Compound I | SAHA+Compound I |
| K562R | 0.9±0.4 | 6.8±3.5 | 17.6±4.5 | 65.3±5.2 |
| LAMA 84R | 2.2±0.9 | 10.3±3.4 | 14.8±4.7 | 66.2±6.1 |

LAMA 84-R cells display approximately a 10-fold higher Compound I I.C.₅₀ values than their sensitive counterparts (e.g., 2.1 vs 0.22 µM; data not shown). Western blots demonstrating the increase in Bcr/Abl protein levels for each cell line, are shown in the insets. It can be seen that co-administration of Compound I (1.0 or 1.25 µM) for 48 hr, which results in only modest lethality in either cell line, with a minimally toxic concentration of SAHA (i.e., 1.0 or 2.0 :M) induced cell death in the majority (e.g. ~66%) of K562R and LAMA 84-R cells. Exposure of sensitive K562 and LAMA 84 cells to these Compound I concentrations for 48 hr induced cell death in virtually 100% of cells (data not shown). Essentially identical results are obtained when cells are exposed to Compound I in combination with SB (data not shown). Such results indicate that co-administration of Compound I with HDIs effectively increases cell death in Compound I-resistant Bcr/Abl⁺ cells, at least in those displaying increased expression of the Bcr/Abl protein.

Finally, to assess the functional contribution of dysregulation of the Raf/MEK/MAP kinase axis to synergistic interactions between Compound I and HDIs in Bcr/Abl+ cells, K562 cells are transiently transfected with a vector expressing either GFP alone or a constitutively active MEK1/2/GFP fusion protein (Table 8).

**Table 8: Purified populations (e.g., >95%) expressing GFP are isolated using a Cytomation MoFLO cell sorter as described in Methods. Untransfected K562 cells displayed 91% viability and 0.01 % GFP expression; K562 cells transfected with GFP alone; sorted cells displayed 95 % viability and 95% GFP expression; K562 cells transfected with GFP/constitutively active MEK1/2 fusion cDNA; sorted cells exhibited 95% viability and 96% GFP-expression. Sorted cells transfected with either GFP alone or the GFP/constitutively active MEK1/2 fusion cDNA are cultured in drug-free medium for 5 hr, and then exposed to 2 .0 µM SAHA ± 250 nM Compound I for 24 hr, after which apoptosis is monitored by examining Wright Giemsa-stained cytospin preparations as described above. Values represent the means ± S.D. for two separate determinations. * = significantly less than values for cells transfected with GFP alone; P < 0.05; ** = P < 0.01. At the end of this period, the extent of apoptosis is monitored as described above. Cells transfected with the constitutively active MEK1/2 are modestly but significantly more resistant to Compound I-mediated lethality than GFP alone controls (P<0.05), consistent with earlier reports demonstrating potentiation of Compound I-induced apoptosis by pharmacologic MEK1/2 inhibitors. Moreover, transiently transfection of cells with mutant MEK1/2 very significantly protects cells from the lethality of the SAHA/Compound I regimen (P<0.01). These findings suggest that dysregulation of the Raf/MEK/MAP kinase cascade in K562 cells exposed to Compound I in conjunction with HDIs plays a significant functional role in enhanced lethality.**

| | GFP | GFP/MEK |
|---|---|---|
| control | 12.1±1.8 | 11.5±1.9 |
| SAHA | 22.5±2.6 | 19.8±2.1 |
| Compound I | 23.8±2.8 | 14.7±2.2 |
| Compound I +SAHA | 68.2±4.6 | 38.4±3.1 |

The results of the present study indicate that co-administration of the Bcr/Abl kinase inhibitor Compound I with clinically relevant HDIs results in a dramatic increase in mitochondrial damage and apoptosis in Bcr/Abl+ human leukemia cells. It has long been known that in human leukemia cells, HDIs, presumably by promoting chromatin relaxation, permit the transcriptional activation of genes involved in the differentiation process. In this regard, HDIs such as SB have been shown to induce erythroid maturation in Bcr/Abl⁺ cell lines such as K562. More recently, attention has focused on the ability of HDIs, particularly the newer generation of compounds, to trigger an apoptotic rather than a maturation program in human leukemia cells. The factors which determine whether an HDI induces cell death versus differentiation in such cells have not been fully elucidated, but the possibility that generation of reactive oxygen species play a role in this process has been suggested. In any case, K562 cells, perhaps due to a generic resistance to apoptosis conferred by constitutive activation of the Bcr/Abl kinase and its downstream cytoprotective targets, are relatively insensitive to HDI-mediated cell death. There are several possible explanations for the finding that co-administration of HDIs and Compound I results in a marked lowering the apoptotic threshold in these Bcr/Abl+ cells. For example, Compound I, by interfering with the anti-apoptotic actions of one or more Bcr/Abl downstream cytoprotective targets, may potentiate the capacity of HDIs to trigger the cell death cascade. Conversely, perturbations in various signaling and cell cycle regulatory pathways induced by HDIs may, in conjunction with those triggered by Compound I, result in amplification of mitochondrial injury and apoptosis. An additional possibility is that Compound I, which has been reported to induce maturation in Bcr/Abl+ cells, may, when combined with HDIs, initiate conflicting signals that result in apoptosis rather than differentiation. In this regard, the finding that dysregulation of leukemic cell maturation represents a potent apoptotic stimulus is well documented. As these mechanisms are not mutually exclusive, the possibility that more than one of them contributes to the marked increase in cell death cannot be excluded.

Several lines of evidence support the notion that interruption of the Raf/MEK/MAP kinase cascade in Bcr/Abl₊ cells by HDIs contributes to the marked induction of apoptosis by the Compound I/HDI regimen. Previous studies have implicated perturbations in MEK/MAP kinase in HDI-mediated differentiation-induction in Bcr/Abl⁺ cells, although differences in the nature of such perturbations have been reported. For example, Rivero reported early activation of ERK in K562 cells exposed to sodium butyrat, whereas Witt et al., described a correlation between butyrate-induced differentiation in K562 cells and inhibition of ERK. Such disparate results may reflect subline-specific differences or, alternatively, a biphasic temporal pattern of ERK activation/down-regulation following butyrate exposure. In this regard, Compound I, which opposes ERK activation, at least at early intervals, has also been shown to promote K562 cell maturation. In accord with these findings, we have also observed early inhibition of ERK activation in Compound I-treated K562 cells, although this was followed by a late rebound to basal levels of activity or above. Significantly, inhibition of MEK/ERK activation in Compound I-treated K562 cells (i.e., by pharmacologic MEK1/2 inhibitors) represents a very potent stimulus for mitochondrial damage and apoptosis [25]. Currently, however, little information is available concerning effects of interruption of the MEK/MAP kinase pathway at upstream sites (e.g., at the level of Raf) in Bcr/Abl⁺ cells. To the best of our knowledge, downregulation of Raf by HDIs in Bcr/Abl⁺ cells has not previously been described. Taken together, these findings are compatible with the concepts that a) down-regulation of the Raf/MEK/ERK axis modifies the maturation program of HDI-treated cells, thereby promoting apoptosis; or b) disruption of the Raf/MEK/ERK cytoprotective pathway lowers the threshold for HDI-mediated cell death. In support of the latter possibility, we have recently observed that pharmacologic MEK1/2/ERK blockade (e.g., by agents such as U0126) results in a marked potentiation of apoptosis in K562 cells exposed to HDIs (C. Yu and S. Grant, unpublished data).

Combined treatment with Compound I and HDIs also resulted in a striking increase in activation of the stress-related kinase JNK. Although exceptions exist, activation of stress-related kinases such as JNK and p38 MAPK generally favors cell death, whereas activation of MEK/MAP kinase exerts cytoprotective effects. In fact, the ratio of the net outputs of the JNK and MAP kinase cascades has been shown to play a key role in survival/cell death decisions. It is therefore tempting to speculate that the dramatic shift from MEK/MAP kinase to JNK signaling in Bcr/Abl₊ cells exposed to the combination of Compound I and HDIs contributed to the marked potentiation of apoptosis.

In addition to disruption of the MEK1/2/ERK pathway, dysregulation of the CDKI p21^{CIP1} could also play a role in synergistic interactions between Compound I and HDIs in Bcr/Abl+ cells. For example, interference with p21^{CIP1} induction (e.g., in cells expressing an antisense construct) or in cells exposed to the CDK inhibitor flavopiridol), has been shown to promote leukemic cell apoptosis following treatment with several differentiation-inducing agents, including PMA, bryostatin 1, and most recently, HDIs including butyrate and SAHA. The basis for this phenomenon is not known with certainty, but may be related to the ability of p21^{CIP1} to bind to and inhibit caspase-3. The observations that acetylation of histones by HDIs specifically activates the p21^{CIP1} promoter, and that p21^{CIP1} is regularly induced by HDIs, particularly in leukemic cells undergoing maturation, suggest that increased expression of this CDKI plays an important role in HDI-mediated maturation. The mechanism by which Compound I blocks p21^{CIP1} induction in HDI-treated cells is not clear, but could stem from disruption of the Raf/MEK/ERK axis, which is known to operate upstream of p21^{CIP1}. Alternatively, Compound I administration, particularly when combined with HDIs, could interfere with the Akt pathway, a downstream target of Bcr/Abl that has also been implicated in the regulation of p21^{CIP1}. The relative contributions, if any, of HDI-associated down-regulation of Raf/MEK/ERK versus Compound 1-mediated interference with p21^{CIP1} induction in synergistic interactions between HDIs and Compound I in Bcr/Abl⁺ cells remain to be defined.

The potentiation of Compound I lethality by HDIs in Compound I-resistant K562 and LAMA 84 cells was similar if not greater than that which we have previously observed in the case of combination regimens involving pharmacologic MEK1/2 inhibitors or, more recently, the CDK inhibitor flavopiridol. Resistance to Compound I can potentially result from multiple factors, including diminished cellular uptake, amplification of *bcr*/*abl* and increased Bcr/Abl protein expression, pharmacokinetic factors, and mutations in the Bcr/Abl kinase domain. For reasons that are unclear, increased expression of Bcr/Abl is the most common mechanism of resistance in cultured cell lines, including those isolated in our laboratory [25,26]. However, in cells obtained from CML patients who have developed in vivo resistance to Compound I, increased Bcr/Abl expression is less frequently observed than mutations in the Bcr/Abl kinase domain. Of these, mutations at the Bcr/Abl kinase contact site (e.g., T315 and Y253) have been the most widely reported. In addition, Corbin et al., have recently employed site-directed mutagenesis to identify other mutations in the Bcr/Abl kinase domain that reduce the inhibitory effects of Compound I, and which could potentially be clinically relevant. The ability of Compound I/HDI regimens to induce apoptosis in otherwise resistant K562 or LAMA 84 cells suggests that this strategy either circumvents the effects of increased Bcr/Abl expression, or, alternatively, acts through pathways that operate downstream or independently of Bcr/Abl. While such a strategy may be effective in cells that display upregulation of Bcr/Abl, it remains to be determined whether it would prove active in cells expressing Bcr/Abl mutations conferring resistance to Compound I. In this regard, the ability of the Compound I/HDI regimen to trigger down-regulation of Bcr/Abl may be relevant, as single amino acid substitutions in the kinase domain may be unlikely to prevent such a process.

By inducing histone acetylation and uncoiling, HDIs promote the expression of genes involved in the maturation process. Consequently, there has been interest in the use of HDIs to enhance the differentiation-inducing capacity of other agents, particularly in leukemia. For example, the ability of HDIs such as butyrate to overcome leukemic cell resistance to all-trans retinoic acid (ATRA) has recently been reported. Because Compound I is capable of inducing differentiation in Bcr/Abl⁺ cells, although to a limited extent, the possibility exists that co-administration of HDIs might enhance this process. However, the data presented here suggest that synergistic interactions between Compound I and HDIs primarily reflect induction of apoptosis rather than maturation. In view of the recent introduction of several novel HDIs into clinical trials in humans, the concept of combining such agents with Compound I for the treatment of patients with CML and related disorders may be feasible. Accordingly, further efforts to explore this strategy are underway.

**Example 2: Example** 3: Capsules with 4-[(4-methyl-1-piperazin-1-ylmethyl)-N-[4-methyl-3-[[4-(3-pyridinyl)-2-pyrimidinyl]amino]phenyl]benzamide methanesulfonate, β-crystal form Capsules containing 119.5 mg of the compound named in the title (Compound I monomethanesulfonate) corresponding to 100 mg of COMPOUND I (free base) as active substance are prepared in the following composition:

| Composition: | SALT I | 119.5 mg |
|---|---|---|
| | Avicel | 200 mg |
| | PVPPXL | 15 mg |
| | Aerosil | 2 mg |
| | Magnesium stearate | 1.5 mg |
| | | 338 mg |

The capsules are prepared by mixing the components and filling the mixture into hard gelatin capsules, size 1.

## Claims

1. Use of a combination for simultaneous, separate or sequential use which comprises (a) N-(5-[4-(4-methyl-piperazino-methyl)-benzoylamido]-2-methylphenyl]-4-(3-pyridyl)-2-pyrimidine of formula I 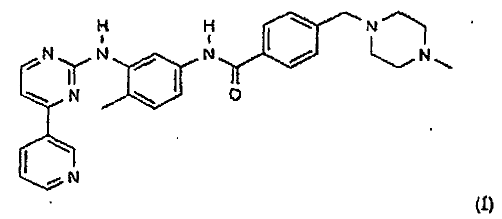 or a pharmaceutically acceptable salt thereof and (b) at least one histone dencetylase inhibitor in free form of in a pharmaceutically acceptable salt form thereof, wherein said histone deacetylase inhibitor is selected from the group consisting of:
(1) SAHA;
(2) Compound of formula II 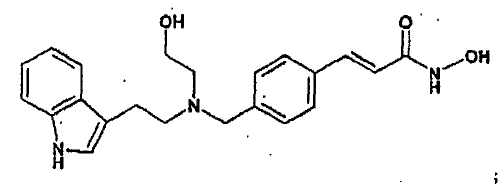 and
(3) Compound of formula III 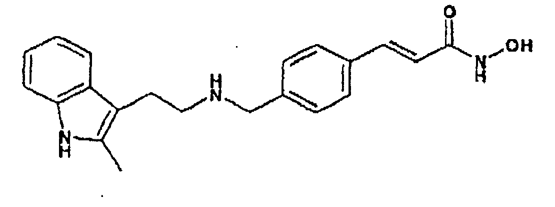 and optionally at least one pharmaceutically acceptable carrier for the preparation of a medicament in the treatment of a leukemia.

2. The use of a combination according to claim 1, wherein said leukemia is a Bcr/Abl+ leukemia.

3. The use of a combination according to any one of the preceding claims, wherein said leukemia is a human Bcr/Abl+ myeloid leukemia.

4. The use of a combination according to any one of the preceding claims wherein said leukemia is a Compound I-resistant leukemia.

5. The use of a combination according to any one of the claims 1-4, wherein said histone deacecylase inhibitor is Compound of formula III or a pharmaceutically acceptable salt thereof

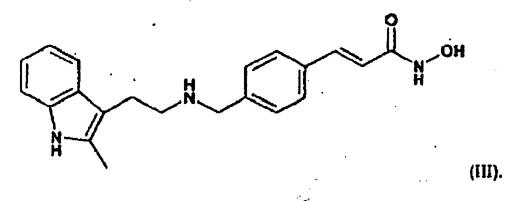

6. The use of a combination according to any one of the preceding claims, wherein said combination is a pharmaceutical fixed combination.

7. A combination for simultaneous, separate or sequential use which comprises (a) N-{5-[4-(4-methyl-piperazino-methyl)-benzoylamido]-2-methylphenyl}-4-(3-pyridyl)-2-pyrimidine of formula I 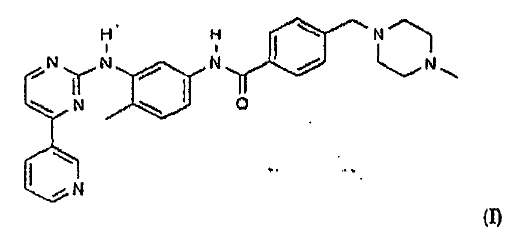 or a pharmaceutically acceptable salt thereof and (b) at least one histone deacetylase inhibitor. in free form of in a pharmaceutically acceptable salt form thereof and optionally at least one pharmaceutically acceptable carrier, wherein (b) is selected from the group consisting of SAHA, Compound of formula II 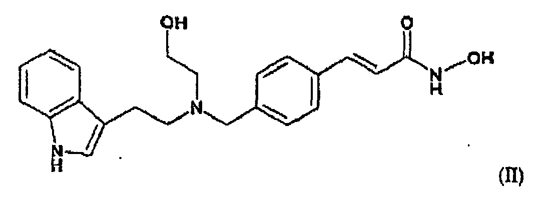 or a pharmaceutically acceptable salt thereof and Compound of formula III or a pharmaceutically acceptable salt thereof.

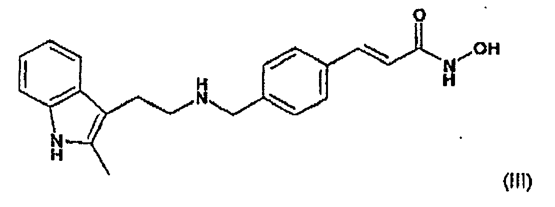

8. A pharmaceutical composition comprising a quantity which is jointly therapeutically effective against a leukemia of a pharmaceutical combination according to claim 7 and at least one pharmaceutically acceptable carrier.

9. A pharmaceutical fixed combination comprising (a) N-{5-[4-(4-methyl-piperazino-methyl)-benzoylamido]-2-methylphenyl)-4-(3-pyridyl)-2-pyrimidine of formula I 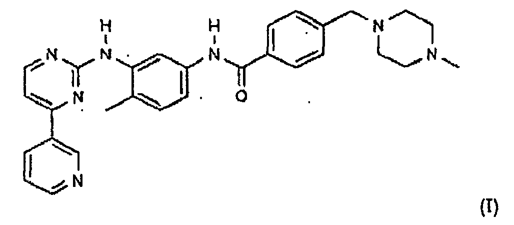 or a pharmaceutically acceptable salt thereof and (b) at least one histone deacetylase inhibitor in free form or in a pharmaceutically acceptable salt form thereof and optionally at least one pharmaceutically acceptable carrier, wherein (b) is selected from the group consisting of SAIIA, Compound of formula II 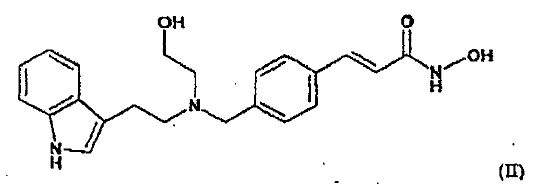 or a pharmaceutically acceptable salt thereof and Compound of formula III or a pharmaceutically acceptable salt thereof.

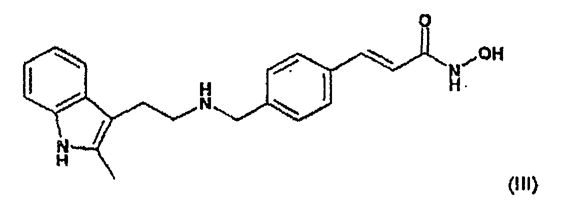

10. The pharmaceutical fixed combination according claim 9, wherein said (b) is Compound of formula III 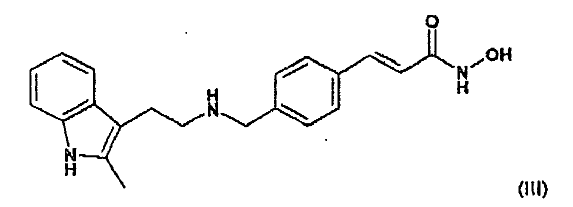 in free form or in a pharmaceutically acceptable sale form thereof and optionally at least one pharmaceutically acceptable carrier.

## Patentansprüche

1. Verwendung einer Kombination zur gleichzeitigen, getrennten oder sequentiellen Verwendung, die (a) N-{5-[4-(4-Methylpiperazinomethyl)-bezoylamido]-2-methylphenyl}-4-(pyridyl)-2-pyrimidin der Formel 1 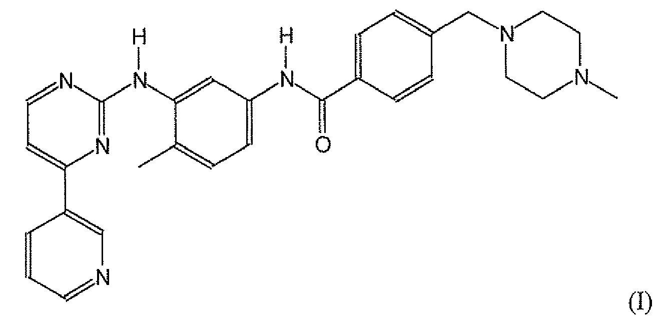 oder ein pharmazeutisch akzeptables Salz davon und (b) mindestens einen Histondeacetylase-Inhibitor in freier Form oder in einer pharmazeutisch akzeptablen Salzform davon umfasst, wobei der Histondeacetylase-Inhibitor ausgewählt ist aus der Gruppe bestehend aus:
(1) SAHA;
(2) der Verbindung nach Formel II 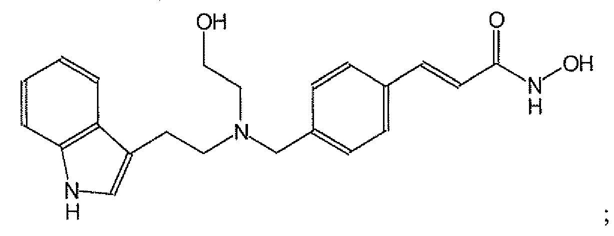 und
(3) der Verbindung nach Formel III 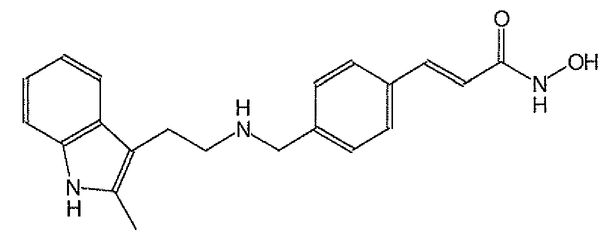
und gegebenenfalls mindestens einen pharmazeutisch akzeptablen Träger zur Herstellung eines Medikaments zur Behandlung einer Leukämie.

2. Verwendung einer Kombination nach Anspruch 1, wobei die Leukämie eine Bcr/Abl+ Leukämie ist.

3. Verwendung einer Kombination nach einem der voranstehenden Ansprüche, wobei die Leukämie eine menschliche Bcr/Abl+ myeloische Leukämie ist.

4. Verwendung einer Kombination nach einem der voranstehenden Ansprüche, wobei die Leukämie eine Verbindung 1-resistent Leukämie ist.

5. Verwendung einer Kombination nach einem der Ansprüche 1 bis 4, wobei der Histondeacetylase-Inhibitor eine Verbindung der Formel III oder ein pharmazeutisch akzeptables Salz davon ist

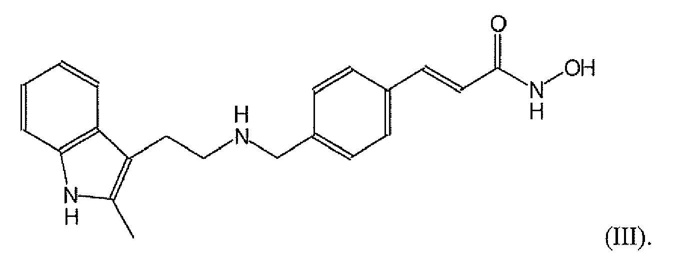

6. Verwendung einer Kombination nach einem der voranstehenden Ansprüche, wobei die Kombination eine pharmazeutische fixierte Kombination ist.

7. Kombination zur gleichzeitigen, getrennten oder sequentiellen Verwendung, umfassend (a) N- {5-[4-(4-Methylpiperazinomethyl)-bezoylamido]-2-methylphenyl}-4-(pyridyl)-2-pyrimidin der Formel I 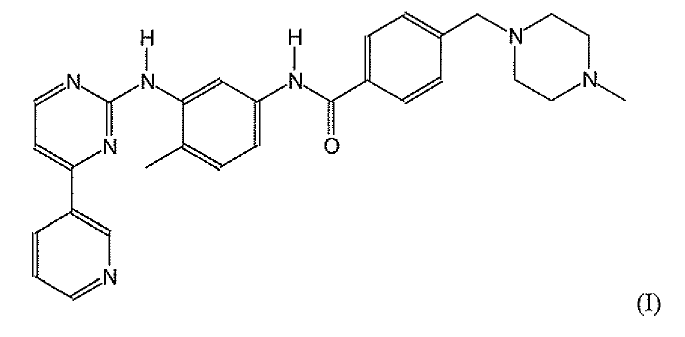 oder ein pharmazeutisch akzeptables Salz davon und (b) mindestens einen Histondeacetylase-Inhibitor in freier Form oder in einer pharmazeutisch akzeptablen Salzform davon und gegebenenfalls mindestens einen pharmazeutisch akzeptablen Träger, wobei (b) ausgewählt ist aus der Gruppe bestehend aus SAHA; der Verbindung nach Formel II 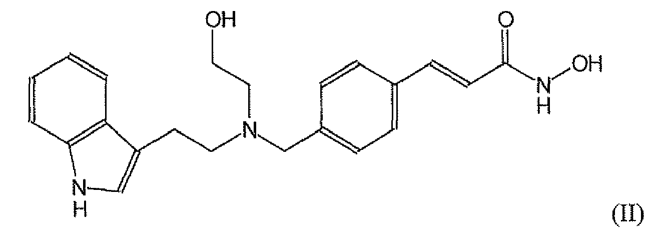 oder einem pharmazeutisch akzeptablen Salz davon und der Verbindung nach Formel III oder einem pharmazeutisch akzeptablen Salz davon

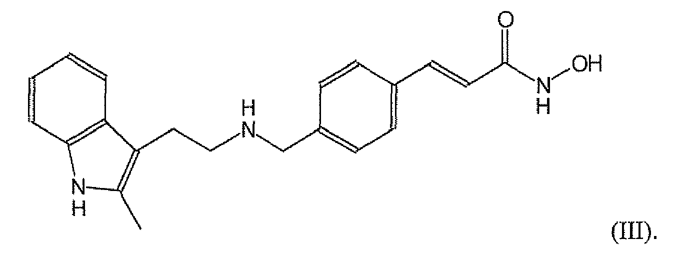

8. Pharmazeutische Zusammensetzung umfassend eine Menge, die gemeinsam therapeutisch gegen eine Leukämie effektiv ist, einer pharmazeutischen Kombination nach Anspruch 7 und mindestens einem pharmazeutisch akzeptablen Träger.

9. Pharmazeutische fixierte Kombination umfassend (a) N-{5-[4-(4-Methylpiperazinomethyl)-bezoylamido]-2-methylphenyl) -4-(pyridyl)-2-pyrimidin der Formel I 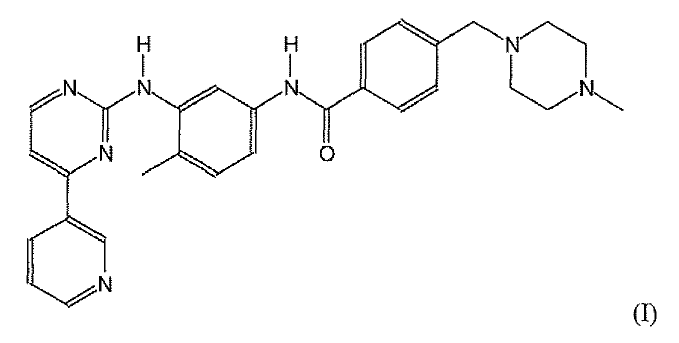 oder ein pharmazeutisch akzeptables Salz davon und (b) mindestens einen Histondeacetylase-Inhibitor in freier Form oder in einer pharmazeutisch akzeptablen Salzform davon und gegebenenfalls mindestens einen pharmazeutisch akzeptablen Träger, wobei (b) ausgewählt ist aus der Gruppe bestehend aus SAHA, der Verbindung nach Formel II 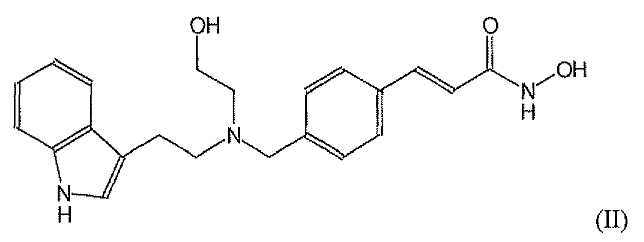 oder einem pharmazeutisch akzeptablen Salz davon und der Verbindung nach Formel III oder einem pharmazeutisch akzeptablen Salz davon

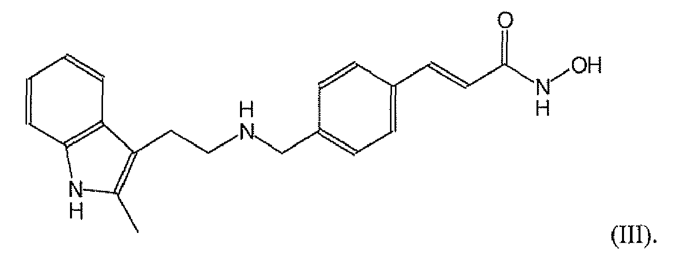

10. Pharmazeutische fixierte Kombination nach Anspruch 9, wobei (b) die Verbindung nach Formel III 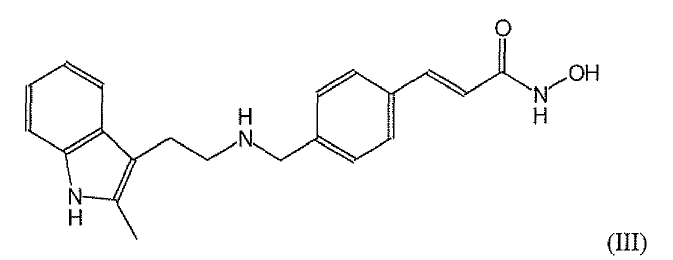 in freier Form oder in einer pharmazeutisch akzeptablen Salzform davon ist und gegebenenfalls mindestens ein pharmazeutisch akzeptabler Träger.

## Revendications

1. Utilisation d'une combinaison, à utiliser de façon simultanée, séparée ou séquentielle, qui comprend (a) N-[5-[4-(4-méthyl-pipérazino-méthyl)-benzoylamido]-2-méthylphényl]-4-(3-pyridyl)-2-pyrimidine de la formule I 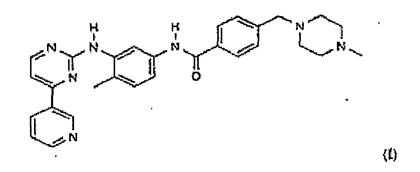 ou un sel pharmaceutiquement acceptable de celle-ci et (b) au moins un inhibiteur de l'histone déacétylase sous une forme libre ou sous la forme d'un sel pharmaceutiquement acceptable de celui-ci, où ledit inhibiteur de l'histone déacétylase est choisi du groupe constitué de :
(1) SAHA ;
(2) le composé de la formule II 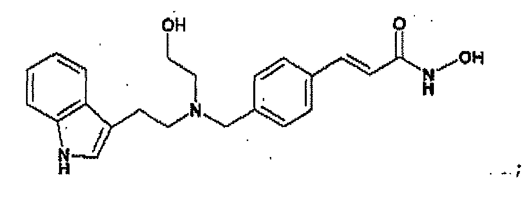 et
(3) le composé de la formule III 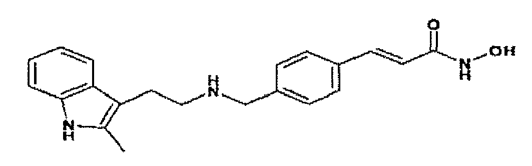 et optionnellement au moins un porteur pharmaceutiquement acceptable pour la préparation d'un médicament pour le traitement de la leucémie.

2. Utilisation d'une combinaison selon la revendication 1, dans laquelle ladite leucémie est une leucémie Bcr/Abl*.

3. Utilisation d'une combinaison selon l'une quelconque des revendications précédentes, dans laquelle ladite leucémie est une leucémie myéloïde humaine Bcr/Abl*.

4. Utilisation d'une combinaison selon l'une quelconque des revendications précédentes, dans laquelle ladite leucémie est une leucémie résistante au composé I.

5. Utilisation d'une combinaison selon l'une quelconque des revendications 1-4, dans laquelle ledit inhibiteur de l'histone déacétylase est un composé de la formule III ou un sel pharmaceutiquement acceptable de celui-ci

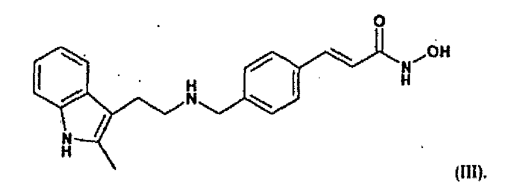

6. Utilisation d'une combinaison selon l'une quelconque des revendications précédentes, dans laquelle ladite combinaison est une combinaison pharmaceutique fixe.

7. Combinaison pour une utilisation simultanée, séparée ou séquentielle qui comprend (a) N-[5-[4-(4-méthyl-pipérazino-méthyl)-benzoylamido]-2-méthylphényl]-4-(3-pyridyl)-2-pyrimidine de la formule I 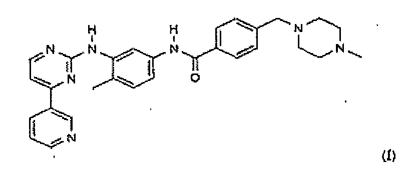 ou un sel pharmaceutiquement acceptable de celle-ci et (b) au moins un inhibiteur de l'histone déacétylase sous une forme libre ou sous forme d'un sel pharmaceutiquement acceptable de celui-ci et optionnellement au moins un porteur pharmaceutiquement acceptable, où (b) est choisi du groupe constitué de SAHA, du composé de la formule II 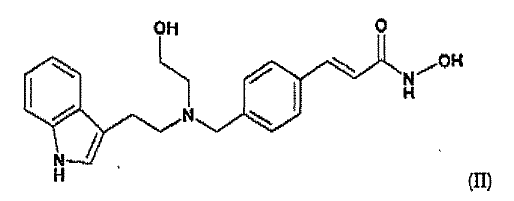 ou un sel pharmaceutiquement acceptable de celui-ci et un composé de la formule III ou un sel pharmaceutiquement acceptable de celui-ci.

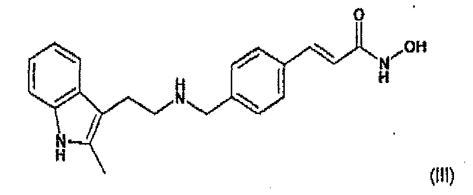

8. Composition pharmaceutique comprenant une quantité qui est conjointement thérapeutiquement efficace contre une leucémie d'une combinaison pharmaceutique selon la revendication 7 et au moins un porteur pharmaceutiquement acceptable.

9. Combinaison pharmaceutique fixe comprenant (a) N-[5-[4-(4-méthyl-pipérazino-méthyl)-benzoylamido]-2-méthylphényl]-4-(3-pyridyl)-2-pyrimidine de la formule I 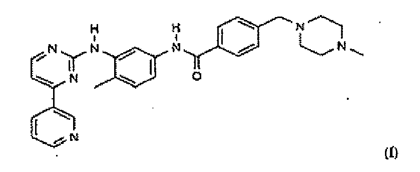 ou un sel pharmaceutiquement acceptable de celle-ci et (b) au moins un inhibiteur de l'histone déacétylase sous une forme libre ou sous forme d'un sel pharmaceutiquement acceptable de celui-ci et optionnellement au moins un porteur pharmaceutiquement acceptable, où (b) est choisi du groupe constitué de SAHA, du composé de la formule II 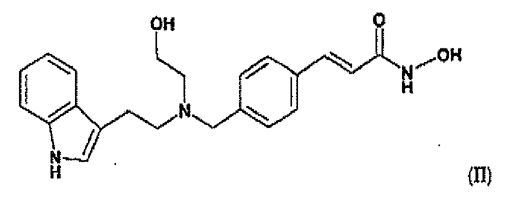 ou un sel pharmaceutiquement acceptable de celui-ci et un composé de la formule III ou un sel pharmaceutiquement acceptable de celui-ci.

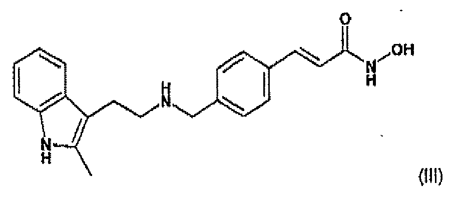

10. Combinaison pharmaceutique fixe selon la revendication 9, dans laquelle ledit (b) est le composé de la formule III 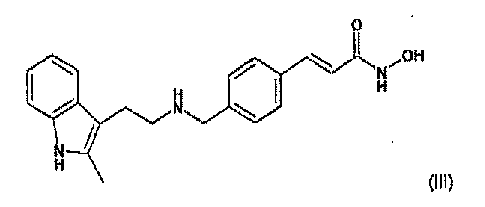 sous une forme libre ou sous forme de sel pharmaceutiquement acceptable de celui-ci et optionellement au moins un porteur pharmaceutiquement acceptable.
